(19) Europäisches Patentamt
European Patent Office
Office européen des brevets

(11) Publication number: 0 130 482 B1

(12) # EUROPEAN PATENT SPECIFICATION

(45) Date of publication of patent specification: 28.12.88

(21) Application number: 84107104.6

(22) Date of filing: 20.06.84

(51) Int. Cl.[4]: C 07 C 87/38, A 61 K 31/28, C 07 D 307/00, C 07 D 205/04, C 07 H 23/00, C 07 C 61/00, C 07 C 62/32, C 07 C 101/00, C 07 C 143/08, C 07 C 147/00, C 07 C 161/00

(54) **Diaminocyclohexane platinum complexes, process for preparing same and pharmaceutical compositions containing same.**

(30) Priority: 20.06.83 US 505965

(43) Date of publication of application: 09.01.85 Bulletin 85/02

(45) Publication of the grant of the patent: 28.12.88 Bulletin 88/52

(84) Designated Contracting States: BE CH DE FR GB IT LI LU NL

(56) References cited:
EP-A-0 026 813
EP-A-0 055 300
DD-A- 158 777
DE-A-3 022 957
US-A-4 140 707
US-A-4 169 846
US-A-4 256 652
US-A-4 322 362

(73) Proprietor: Research Corporation
405 Lexington Avenue
New York, N.Y. 10174 (US)

(72) Inventor: Brown, Davis B.
Deceased (US)
Inventor: Khokhar, Abdul R.
51 1/2 Latham Court
Burlington Vermont (US)
Inventor: Hacker, Miles P.
153 Oak Hill Road
Williston Vermont (US)
Inventor: McCommack, John J.
60 Bilodeau Court
Burlington Vermont (US)

(74) Representative: Brauns, Hans-Adolf, Dr. rer. nat. et al
Hoffmann, Eitle & Partner, Patentanwälte
Arabellastrasse 4
D-8000 Munich 81 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).



Courier Press, Leamington Spa, England.

**Description**

The present invention relates to certain novel 1,2-diaminocyclohexane platinum complexes, pharmaceutical compositions for use in the treatment of tumors and a process for preparing the complexes.

Recently, certain platinum complexes have been shown by Rosenberg et al and others to be highly active anti-tumor agents (see U.S. Patents 4,177,263 and 4,140,707). For example, the complex *cis*-dichlorodiammine-platinum-II or "cisplatin" is the chemotherapeutic agent of choice in the treatment of many and varied tumors.

There are several drawbacks, however, associated with the use of the platinum complexes to treat tumors. Generally, the platinum complexes have a relatively low solubility in water thereby rendering it difficult to formulate a composition which can effectively deliver the reagent to the site of the tumor in the body.

Moreover, many of the platinum complexes are highly nephrotoxic thereby further restricting their use in the absence of precautionary measures to avoid damage to the kidneys when administered to animals afflicted with tumors.

Recently, considerable activity has centered on the use of 1,2-diaminocyclohexane complexes of platinum as anti-tumor agents. See, for example, U.S. Patents Nos. 3,892,790; 3,904,663; 4,115,418; 4,140,707; 4,169,846; 4,175,133; 4,228,090 and 4,256,652 and German Offenlegungschrift 30 22 917.

Although the complexes described therein possess anti-tumor activity, virtually all are difficultly soluble in water and are also relatively highly nephrotoxic.

German Offenlegungschrift 30 22 957 describes 1,2-diaminocyclohexane/ascorbate complexes of platinum, useful as anti-tumor agents, which comprise mixtures of complexes having varying ratios of ascorbate ion to platinum. Thus, the publication discloses complexes of the formula:

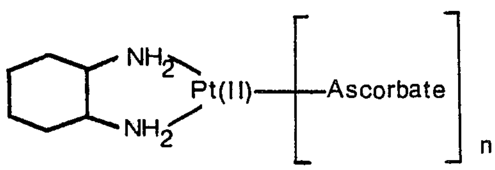

wherein n varies from 1.2 to 1.5. The complexes are described as poorly water soluble.

The hereinbelow described method enables the production of complexes of DACH-platinum complexes of the following formulae which contain stoichiometric ratios of anion to platinum:

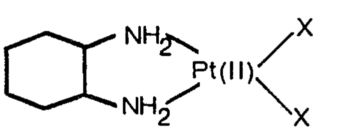 or, 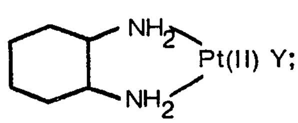

wherein:

X is a monovalent anion selected from the group consisting of monosaccharate, saccharate-4-lactone, shikimate, isethionate, 2-aminoethylsulfate, azetidinecarboxylate, proline, hydroxyproline, pipecolinate, cyclopropanecarboxylate, cyclobutanecarboxylate, cyclopentanecarboxylate, cyclopentenecarboxylate, cyclohexanecarboxylate, cyclohexenecarboxylate, bicine, glycine, 2-aminoethanesulfonate, 2-chloroethanesulfonate, and

Y is a divalent anion selected from the group consisting of iminodiacetate, isocitratelactone, furanedicarboxylate, cyclopropane-1, 1-dicarboxylate and isocitratomonoethyl esther, N-methyliminodiacetate, N-(2-hydroxyethyl)-iminodiacetate, N-benzyliminodiacetate, N-phenyliminodiacetate, N-(2-acetamido)-iminodiacetate, cyclohexane-1,1-diacetate, trans-1,2-cyclopropanedicarboxylate, trans-1,2-cyclobutanedicarboxylate, trans-1,2-cyclopentanedicarboxylate and trans-1,2-cyclohexanedicarboxylate.

The method of the invention for preparing the above-described complexes comprises:

a) reacting a water-soluble haloplatinate(II) in an aqueous medium with DACH to produce a di-halo-(DACH)-platinum(II);

b) reacting said product of step a) with a soluble sulfate salt in an aqueous medium to produce sulfato(DACH)-platinum(II);

c) reacting the product of step b) with a soluble salt of X or Y to produce said complex of the above formula, and

d) recovering said complex.

The present invention also provides a pharmaceutical composition in unit dosage form suitable for administration to an animal afflicted with tumor cells sensitive to a platinum complex of the above formula comprising a therapeutically anti-tumor effective amount of the platinum complex and a pharmaceutically acceptable carrier therefor.

The invention also provides a method for the treatment of an animal afflicted with tumor cells sensitive to a platinum complex of the above formula comprising administering to the animal a therapeutically, anti-tumor effective amount of the platinum complex.

Detailed Description of the Invention

The complexes of the invention have the following structural formulae:

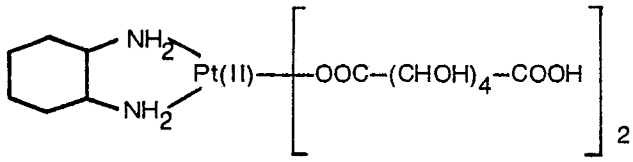

[bis-monosaccharato]

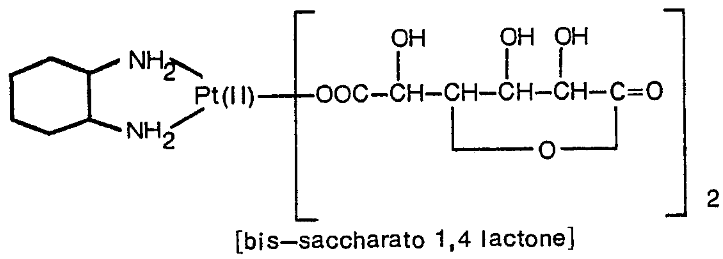

[bis—saccharato 1,4 lactone]

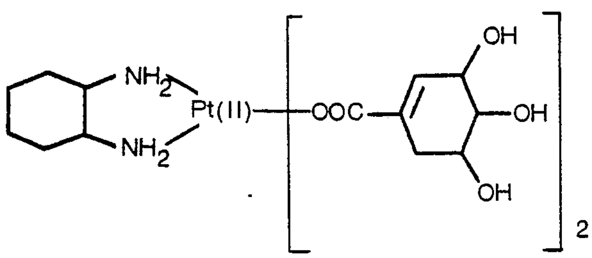

[bis—shikimato]

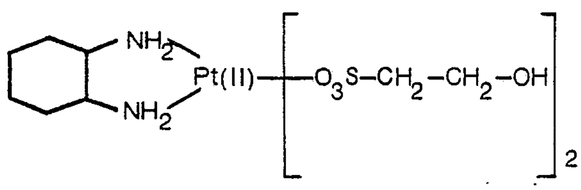

[bis—isethionato]

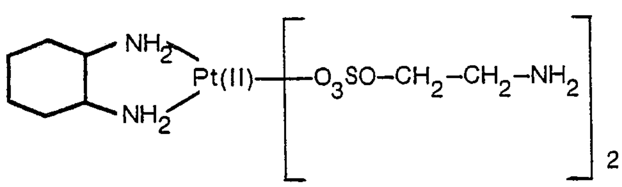

[bis—2—aminoethylsulfato]

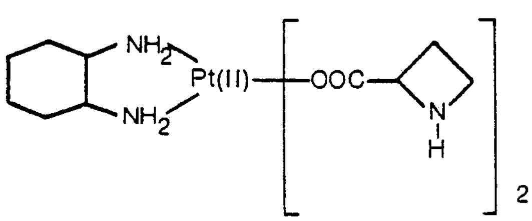

[bis—azetidinecarboxylato]

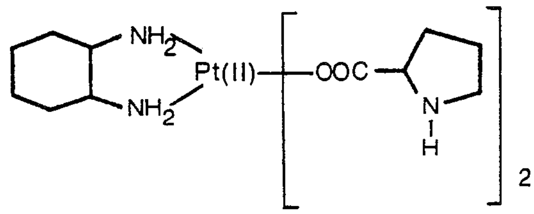

[bis–prolinato]

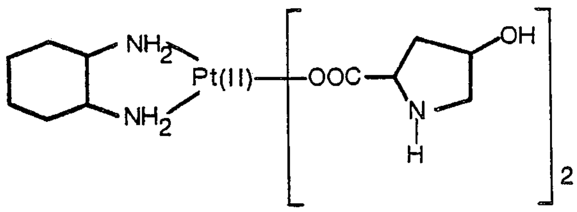

[bis–hydroxyprolinato]

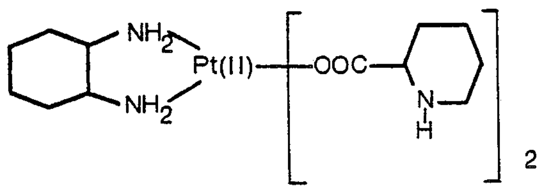

[bis–pipecolinato]

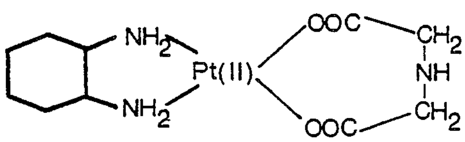

[Iminodiacetato]

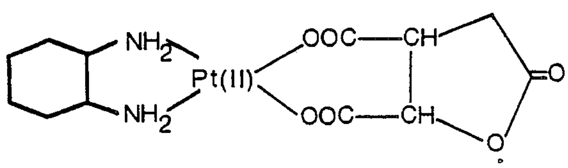

[isocitratolactone]

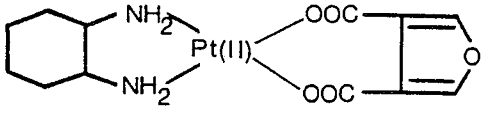

[furanedicarboxylate]

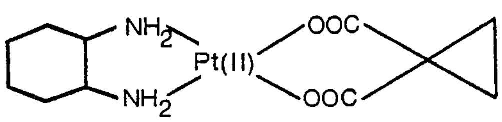

[cyclopropanedicarboxylato–]

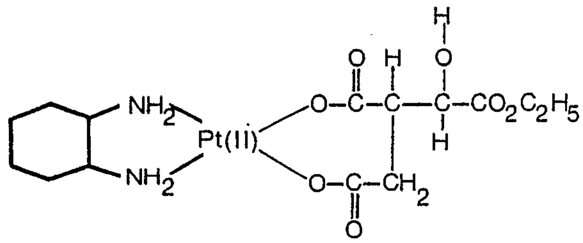

[isocitratomonoethylester]

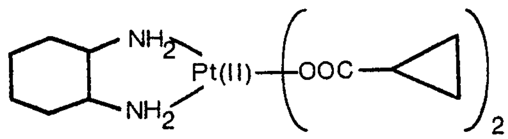

[bis–cyclopropanecarboxylato]

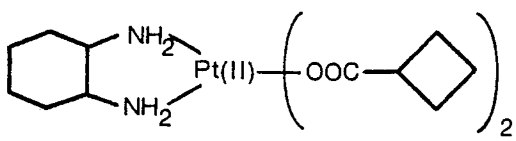

[bis–cyclobutanecarboxylato]

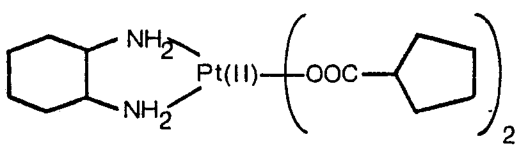

[bis–cyclopentanecarboxylato]

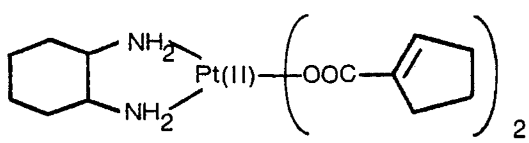

[bis-cyclopentenecarboxylato]

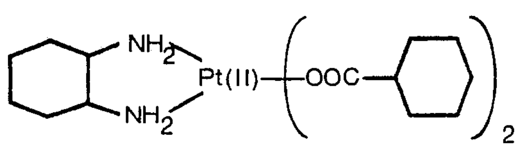

[bis–cyclohexanecarboxylato]

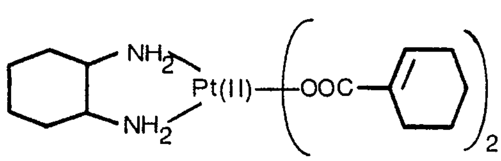

[bis-cyclohexenecarboxylato]

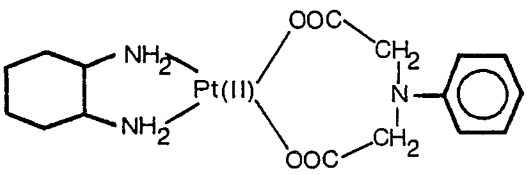

[N–phenyliminodiacetato]

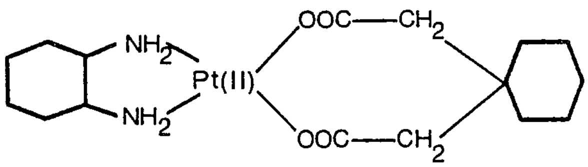

[cyclohexane–1,1–diacetato]

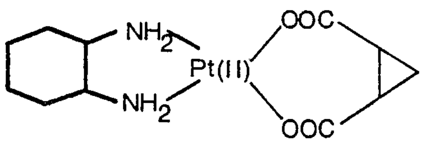

[1,2–cyclopropanedicarboxylato]

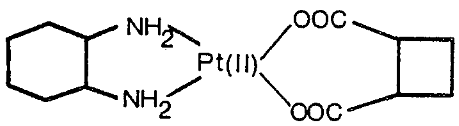

[trans–1,2–cyclobutanedicarboxylato]

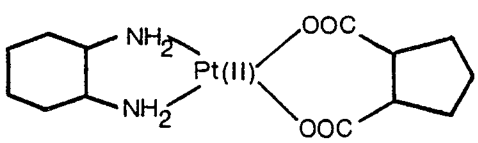

[trans–1,2–cyclopentanedicarboxylato]

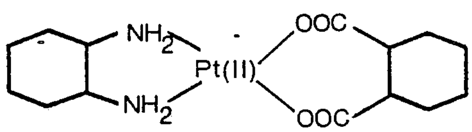

[trans–1,2–cyclohexanedicarboxylato]

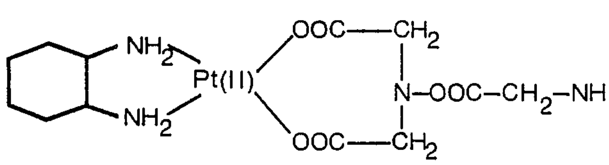

[N-(2-acetamido)-iminodiacetato]

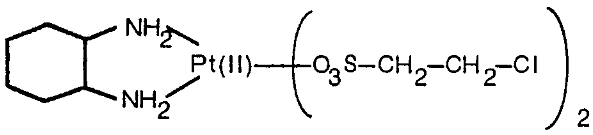

[bis-2-chloroethanesulfonato]

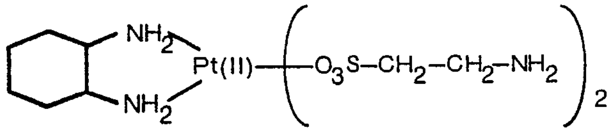

[bis-2-aminoethanesulfonato]

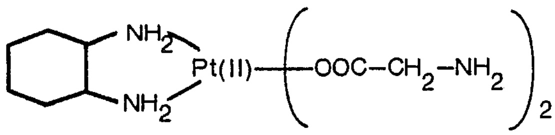

[bis-glycinato]

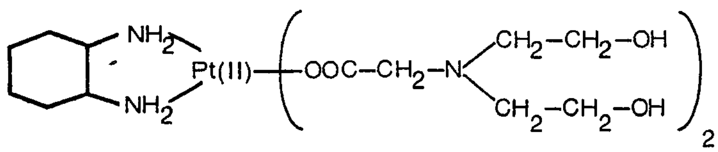

[bis-bicinato]

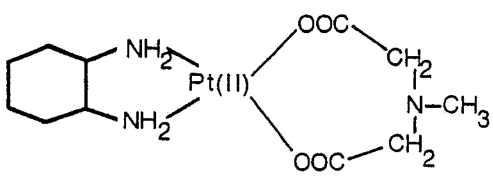

[N-methyliminodiacetato]

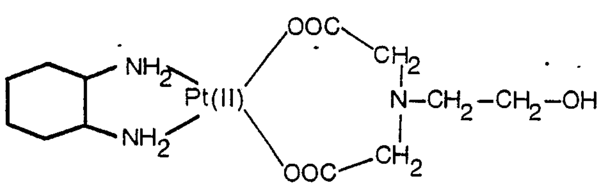

[N-(2-hydroxyethyl)iminodiacetato]

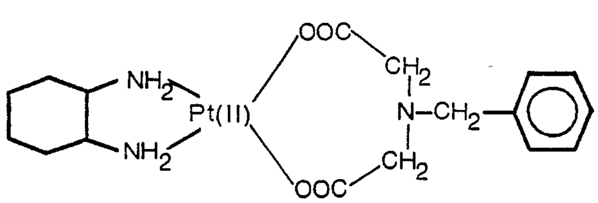

[N-benzyliminodiacetato]

The stoichiometric mono- and bis- anion-(DACH) platinum complexes of the invention (i.e., containing one divalent anion or two monovalent anions per molecule of platinum) may be prepared by reacting any suitable water-soluble haloplatinate (i.e., $K_2PtCl_4$) with DACH to produce the intermediate, di-halo-(DACH)platinum(II). The reaction is preferably effected in water at room temperature for a time sufficient to drive the reaction to completion, generally from about 6 to 8 hours.

The intermediate di-halo complex is normally a yellow solid precipitate which is advantageously isolated from the reaction mixture by filtering, followed by washing with water, methanol and acetone. Finally, the yellow solid is dried, preferably under vacuum or is left in solution and immediately reacted therein with a sulfate. It is preferred to utilize a sulfate of a metal or cation the halo salt of which is insoluble in water to facilitate isolation of the product. Thus, where the di-halo (DACH) platinum is the di-chloro complex, it is preferred to employ $Ag_2SO_4$ thereby facilitating removal of the insoluble by-product, AgCl. The reaction is preferably carried out in an aqueous medium such as water or in the reaction medium from the first step of the method at room temperature. The reaction generally goes to completion in from about 18 to about 24 hours.

Following removal of the insoluble by-product halide, the intermediate sulfato (DACH-platinum(II)) is isolated by evaporating the yellow filtrate to dryness, e.g., using a rotary evaporator, as a yellow-brown solid which is washed, e.g., with acetone and dried, preferably, in vacuum. Alternatively, the sulfato-DACH-platinum intermediate is left in solution and reacted therein with a soluble salt of the appropriate anion to yield the platinum complex of the invention.

It is preferred to utilize a metal or other cation salt of the anion whose by-product sulfate salt is insoluble in water to facilitate isolation of the platinum complex. For example, the use of a barium salt of the anion yields barium sulfate as a by-product which is readily removable from the reaction medium by filtration.

The reaction is preferably conducted in an aqueous medium at about room temperature for a time sufficient to drive the reaction to completion, generally from about 0.3 to about 0.5 hours.

The platinum complex may be isolated from the reaction medium by filtering the by-product barium sulfate. The yellow filtrate is evaporated to dryness at 45—50°C under reduced pressure, e.g., using a rotary evaporator. The yellow brown solid is then dried, e.g., over $P_2O_5$ under vacuum.

The method of invention is illustrated by the following non-limiting examples. The products of preparative examples 1—6, 14, 15 and 16 are summarized in Table 1.

TABLE 1

| Compound No | Compound | Example No. |
|---|---|---|
| 1 | cis-bis-D-monosaccharato(DACH)Pt(II).3$H_2O$ | 1 |
| 2 | cis-bis-D-saccharato-1,4-lactone(DACH)Pt(II) | 2 |
| 3 | cis-bis-shikimato(DACH)Pt(II).2$H_2O$ | 3 |
| 4 | iminodiacetato(DACH)Pt(II).2$H_2O$ | 4 |
| 5 | DL-isocitratolactone(DACH)Pt(II).1$H_2O$ | 5 |
| 6 | cis-bis-isethionato(DACH)Pt(II).2$H_2O$ | 6 |
| 7 | cis-bis-2-aminoethylsulfato(DACH)Pt(II) | 6 |
| 8 | cis-bis-L-azetidinecarboxylato(DACH)Pt(II).1$H_2O$ | 6 |
| 9 | cis-bis-L-prolinato(DACH)Pt(II) | 6 |
| 10 | cis-bis-L-hyroxyprolinato(DACH)Pt(II).1$H_2O$ | 6 |
| 11 | cis-bis-DL-pipecolinato(DACH)Pt(II).1$H_2O$ | 6 |
| 12 | furanedicarboxylato(DACH)Pt(II) | 6 |
| 13 | cyclopropane-1,1-dicarboxylato(DACH)Pt(II).$H_2O$ | 6 |
| 14 | isocitratomonoethylester(DACH)Pt(II).1.5$H_2O$ | 6 |
| 15 | cis-bis-cyclohexanecarboxylato(DACH)Pt(II).1$H_2O$ | 16 |

TABLE 1 (continued)

| Compound No | Compound | Example No. |
|---|---|---|
| 16 | 1,1-cyclohexanediacetato(DACH)Pt(II).1$H_2O$ | 16 |
| 17 | cis-bis-cyclohexenecarboxylato(DACH)Pt(II).1$H_2O$ | 16 |
| 18 | cis-bis-cyclopentanecarboxylato(DACH)Pt(II).1$H_2O$ | 16 |
| 19 | cis-bis-cyclopentenecarboxylato(DACH)Pt(II).2$H_2O$ | 16 |
| 20 | trans-DL-1,2-cyclopentanedicarboxylato(DACH)Pt(II) | 16 |
| 21 | N-Methyliminodiacetato(DACH)Pt(II).1$H_2O$ | 16 |
| 22 | cis-bis-cyclopropanecarboxylato(DACH)Pt(II).1$H_2O$ | 16 |
| 23 | cis-bis-cyclobutanecarboxylato(DACH)Pt(II).1$H_2O$ | 16 |
| 24 | cis-bis-ethanesulfonato(DACH)Pt(II).$H_2O$ | 16 |
| 25 | cis-bis-glycinato(DACH)Pt(II).1$H_2O$ | 16 |
| 26 | cis-bis-chloroethanesulfonato(DACH)Pt(II).1$H_2O$ | 16 |
| 27 | cis-bis-bicinato(DACH)Pt(II) | 16 |
| 28 | Iminodiacetato(trans-1-DACH)Pt(II).2$H_2O$ | 15 |
| 29 | cis-bis-L-prolinato(trans-1-DACH)Pt(II).2$H_2O$ | 14 |
| 30 | N-Phenyliminodiacetato(DACH)Pt(II).2$H_2O$ | 16 |
| 31 | N-Benzyliminodiacetato(DACH)Pt(II) | 16 |
| 32 | N-(2-Hydroxyethyl)-iminodiacetato(DACH)Pt(II).2$H_2O$ | 16 |
| 33 | trans-1,2-cyclopropanedicarboxylato(DACH)Pt(II) | 16 |
| 34 | trans-1,2-cyclobutanedicarboxylato(DACH)Pt(II) | 16 |
| 35 | N-(2-Acetamido)-iminodiacetato(DACH)Pt(II).½$H_2O$ | 16 |
| 36 | trans-1,2-cyclohexanedicarboxylato(DACH)Pt(II) | 16 |

Example 1

Cis-bis-D-monosaccharato(DACH)platinum(II).3$H_2O$

Sulfato(DACH)platinum(II) 1.0 g (2.47 mmole) was dissolved in water (50 ml) and a solution of barium monosaccharate (1.372 g; 2.47 mmole in 80 ml of water) was added thereto. The reaction mixture was stirred at room temperature for 20 minutes. Barium sulfate precipitate was filtered off, and the yellow filtrate was evaporated to dryness at 45—50°C under reduced pressure using a rotary evaporator. A brown yellow solid was obtained, which was then washed with acetone and dried in vacuo.

Yield = 89%. The analytical data for the product is set forth in Table II.

Platinum Analysis — calculated for $C_{18}H_{32}N_2O_{16}$: Pt.3$H_2O$: Pt 24.96%. Found: Pt 24.77%.

Example 2

Cis-bis-D-saccharate-1,4-lactone(DACH)platinum(II)

Sulfato(DACH)platinum(II) 0.101 g was dissolved in 10 ml of water and barium saccharate 1.4 lactone, prepared in situ by the addition of $Ba(OH)_2.8H_2O$ 0.075 g to an aqueous solution of D-saccharic acid 1,4 lactone mono-hydrate 0.11 g was added thereto and the reaction mixture was stirred for 30 minutes at room temperature. Barium sulfate precipitate was filtered off and the yellow filtrate was evaporated to

dryness at 45—50°C under reduced pressure using a rotary evaporator. A brown yellow solid was obtained, which was then washed with methanol and acetone. The product was finally dried in vacuo.

Yield = 82%. The analytical data for the product is set forth in Table II.

Example 3

Cis-bis-shikimato(DACH)platinum(II)

Sulfato(DACH)platinum(II) 0.405 g (1 mmole) was dissolved in water (20 ml) and a solution of barium shikimate (0.0483 g; 1 mmole in 50 ml of water) was added thereto. The reaction mixture was stirred at room temperature for 20 minutes. Barium sulfate precipitate was filtered off, and the yellow filtrate was evaporated to dryness at 45—50°C under reduced pressure using a rotary evaporator. A brown yellow solid was obtained, which was then washed with acetone and dried in vacuo.

Yield = 80%. The analytical data for the product is set forth in Table II.

Platinum Analysis: Calculated for $C_{20}H_{32}N_2O_{10}$ Pt.$2H_2O$, Pt 28.20%. Found: Pt 28.76%.

Example 4

Iminodiacetato(DACH)platinum(II)

Sulfato(DACH)platinum(II) 1.05 g (2.47 mmole) was dissolved in water (50 ml) and a solution of barium iminodiacetate (0.633 g; 2.57 mmole in 50 ml of water) was added thereto. The reaction mixture was stirred at room temperature for 20 minutes. Barium sulfate precipitate was filtered off, and the yellow filtrate was evaporated to dryness at 45—50°C under reduced pressure using a rotary evaporator. A brown yellow solid was obtained, which was then washed with acetone and dried in vacuo.

Yield = 79%. The analytical data for the product is set forth in Table II.

Example 5

DL-Isocitratolactone(DACH)platinum(II)

Sulfato(DACH)platinum(II) 0.1 g (0.25 mmole) was dissolved in water (10 ml) and a solution of barium isocitratolactone (0.766 g; 0.25 mmole in 20 ml of water) was added thereto. The reaction mixture was stirred at room temperature for 20 minutes. Barium sulfate precipitate was filtered off, and the yellow filtrate was evaporated to dryness at 45—50°C under reduced pressure using a rotary evaporator. A brown yellow solid was obtained, which was then washed with acetone and dried in vacuo.

Yield = 75%. The analytical data for the product is set forth in Table II.

Example 6

The other complexes of the invention, i.e., bis-isethionato, bis-2-aminoethylsulfato, bis-azetidinecarboxylato, bis-prolinato, bis-hydroxyprolinato, bis-pipecolinato, furanedicarboxylato, cyclopropane-1,1-dicarboxylato- and isocitratomonoethyl ester were prepared in an analogous manner to the methods of Example 8, using stoichiometric amounts (ca. 1 mmole) sulfato(DACH)platinum(II) and the respective barium salts, i.e., isethionate, 2-aminoethylsulfate, azetidinecarboxylate, proline, hydroxyproline, pipecolinate, furanedicarboxylate, cyclopropane, dicarboxylate and isocitratemonoethyl ester.

It will be understood by those skilled in the art that the various isomeric DACH (i.e., trans-d-, trans-1-, cis-) can be prepared according to the methods of the above examples employing the appropriate DACH isomer. The analytical data for the compounds prepared in Examples 1—13 are set forth in Table II.

TABLE II

| Compound No. | Analytical Data<br>Found (%)<br>C | H | N | Calculated (%)<br>C | H | N |
|---|---|---|---|---|---|---|
| 1 | 27.16 | 4.23 | 3.23 | 27.64 | 4.86 | 3.58 |
| 2 | 29.66 | 4.15 | 3.53 | 29.70 | 3.85 | 3.85 |
| 3 | 34.60 | 5.11 | 3.75 | 34.72 | 5.20 | 4.05 |
| 4 | 25.35 | 4.83 | 8.57 | 25.20 | 4.83 | 8.82 |
| 5 | 28.65 | 4.13 | 5.42 | 28.85 | 4.00 | 5.61 |
| 6 | 19.62 | 4.16 | 4.19 | 20.16 | 4.70 | 4.70 |
| 7 | 20.28 | 4.62 | 8.99 | 20.36 | 4.41 | 9.50 |
| 8 | 31.98 | 5.20 | 10.48 | 31.86 | 4.31 | 10.62 |

TABLE II (continued)

| Compound No. | Analytical Data Found (%) C | H | N | Calculated (%) C | H | N |
|---|---|---|---|---|---|---|
| 9 | 35.63 | 5.77 | 9.87 | 35.73 | 5.58 | 10.42 |
| 10 | 32.75 | 5.57 | 9.39 | 32.69 | 5.11 | 9.53 |
| 11 | 37.30 | 6.21 | 9.46 | 37.03 | 6.17 | 9.70 |
| 12 | 28.84 | 3.53 | 5.33 | 28.80 | 4.00 | 5.60 |
| 13 | 29.37 | 4.44 | 5.50 | 28.94 | 4.60 | 6.14 |
| 14 | 30.10 | 4.46 | 5.32 | 30.32 | 4.87 | 5.05 |

The *in vitro* anti-tumor activities of the complexes of the invention are illustrated by the following examples:

Example 7

Wild type L1210 leukemic cells were grown as a suspension culture in McCoy's 5A medium supplemented with 10% horse serum, glutamine, streptomycin and penicillin at 37°C, 95% relative humidity and 5% $CO_2$. Four ml of cell suspension ($10^5$ cells/ml) are added to culture tubes and the appropriate concentration (0.01, 0.1, 1 or 10 µg/ml final concentration) of drug added to the culture tubes. After 72 hours of incubation, the cell concentration of control and experimental cultures are determined with the aid of a Coulter Counter® Model $ZB_f$ and the percent inhibition calculated.

| Compound Number | $ID_{50}$ (µg/ml) |
|---|---|
| 1 | 2.8 |
| 2 | 3.0 |
| 3 | 2.5 |
| 4 | 4.2 |
| 5 | 1.0 |
| 6 | 0.43 |
| 7 | 4.3 |
| 8 | 3.6 |
| 9 | 4.0 |
| 10 | 2.7 |
| 11 | 0.44 |
| 12 | 1.0 |
| 13 | 0.41 |
| 14 | 2.0 |

The lack of cross-resistance of complexes of the invention to cisdiamminedichloroplatinum(II) is illustrated below.

Example 8

L1210 leukemia cells (L1210/PDD) which are more than 50 fold resistant to cisdiamminedichloroplatinum(II) were grown as suspension cultures in McCoy's SA supplemented with 10% horse serum, glutamine, penicillin and streptomycin at 37°C, 95% relative humidity and 5% $CO_2$. Four ml of cell suspension were added to culture tubes and the appropriate concentration (0.01, 0.1, 1 or 10 µg/ml final drug concentration) was added. After 96 hours the cell concentration of control and experimental cultures were calculated with the aid of a Coulter Counter® Model $ZB_f$ and the percent inhibition calculated.

| Compound Number | $ID_{50}$(µg/ml) L1210/0 | $ID_{50}$(µg/ml) L1210/PDD |
|---|---|---|
| 1 | 2.8 | 0.9 |
| 2 | 3.0 | 0.6 |
| 3 | 2.5 | 1.2 |
| 4 | 4.2 | 1.6 |
| cisdiamminedichloroplatinum(II) | 0.1 | 5.5 |

The anti-tumor activities of the complexes of the invention are illustrated by the following example:

Example 9

$BDF_1$ mice were inoculated intraperitoneally with $10^6$ L1210 cells. About 24 hours after inoculation of the cells, the mice were injected intraperitoneally with varying dosages of complexes of the present invention. Six mice were used for each dosage level in each experiment with an equal number of control mice inoculated with $10^6$ L1210 cells and left untreated with a given complex. The results [%T/C = (survival time of treated animals/survival-time of control animals) × 100] are set forth below. Long term survival signifies that animals were alive 30 days after inoculation with L1210 cells.

| Compound Number | Dose (mg/kg) | %T/C | Long term Survivors |
|---|---|---|---|
| 1 | 12.5 | 120 | — |
| | 25 | 129 | — |
| | 50 | 156 | — |
| | 100 | 151 | — |
| | 200 | Toxic | — |
| 2 | 12.5 | 127 | — |
| | 25 | 138 | — |
| | 50 | 156 | — |
| | 100 | Toxic | |
| 3 | 50 | 140 | — |
| | 100 | 217 | 2/6 |
| 4 | 50 | 163 | — |
| | 100 | 109* | — |
| 5 | 25 | 133 | — |
| | 50 | 145 | — |
| | 100 | Toxic | — |

* Apparent Toxicity

| Compound Number | Dose (mg/kg) | %T/C | Long term Survivors |
|---|---|---|---|
| 6 | 5 | 153 | — |
| | 10 | 180 | — |
| | 20 | 140* | 1/6 |
| | 50 | Toxic | — |

*2/6 animals died of apparent drug toxicity

| Compound Number | Dose (mg/kg) | %T/C | Long term Survivors |
|---|---|---|---|
| 7 | 6.25 | | |
| | 12.5 | In | |
| | 25 | Progress | |
| | 50 | | |
| 8 | 12.5 | 157 | — |
| | 25 | 148 | — |
| | 50 | 161 | — |
| | 100 | Toxic | — |
| 9 | 12.5 | 144 | — |
| | 25 | 165 | — |
| | 50 | 165 | — |
| | 100 | 162* | — |
| | *1 animal died from apparent toxicity | | |
| 10 | 25 | 160 | — |
| | 50 | 170 | — |
| | 100 | Toxic | — |
| 11 | 12.5 | 145 | — |
| | 25 | 180 | — |
| | 50 | Toxic | |
| 12 | 6.25 | 143 | — |
| | 12.5 | 136 | — |
| | 25 | 136 | — |
| | 50 | Toxic | — |

Example 10

The procedure of Example 16 was repeated except that the treated animals were injected with multiple doses of the complex as indicated below:

| Compound Number | Dose (mg/kg) | Day of Administration | %T/C | Long Term Survivors |
|---|---|---|---|---|
| 1 | 25 | 1,5,9 | 206 | — |
| | 50 | 1,5,9 | 225 | 1/6 |
| 3 | 12.5 | 1,5,9 | 139 | |
| | 25 | 1,5,9 | 149 | — |
| | 50 | 1,5,9 | 222 | 1/6 |
| 4 | 25 | 1,5,9 | 199 | — |
| | 50 | 1,5,9 | 220 | 2/6 |
| 5 | 25 | 1,5,9 | 219 | — |
| | 50 | 1,5,9 | 165 | — |

Example 11

Male albino mice were administered a single intraperitoneal injection of the appropriate test compound and were observed daily for signs of toxicity and survival. Fourteen days after treatment all surviving mice were sacrificed and the $LD_{10}$, $LD_{50}$ and $LD_{90}$ were calculated.

| Compound Number | Dose (mg/kg) | Deaths/ Treated |
|---|---|---|
| 1 | 100 | 1/6 |
| | 125 | 1/6 |
| | 158 | 4/6 |
| | 200 | 3/6 |
| | 250 | 6/6 |
| Calculated $LD_{10}$ 115 mg/kg | | |
| Calculated $LD_{50}$ 155 mg/kg | | |
| Calculated $LD_{90}$ 220 mg/kg | | |
| 2 | 85 | 0/6 |
| | 100 | 0/6 |
| | 125 | 3/6 |
| | 158 | 5/6 |
| | 200 | 4/6 |
| Calculated $LD_{10}$ 100 mg/kg | | |
| Calculated $LD_{50}$ 140 mg/kg | | |
| Calculated $LD_{90}$ 185 mg/kg | | |
| 3 | 50 | 0/6 |
| | 100 | 1/6 |
| | 125 | 3/6 |
| | 160 | 5/6 |
| | 200 | 3/6 |
| Calculated $LD_{10}$ 85 mg/kg | | |
| Calculated $LD_{50}$ 130 mg/kg | | |
| Calculated $LD_{90}$ 205 mg/kg | | |
| 4 | 85 | 1/6 |
| | 100 | 3/6 |
| | 125 | 5/6 |
| | 158 | 4/6 |
| | 200 | 6/6 |
| Calculated $LD_{10}$ 80 mg/kg | | |
| Calculated $LD_{50}$ 115 mg/kg | | |
| Calculated $LD_{90}$ 155 mg/kg | | |

The potential nephrotoxicity of selected complexes of the invention are set forth below:

Example 12

Male albino mice were administered a single intraperitoneal injection of either the calculated $LD_{10}$ or $LD_{50}$ of the test compound. Blood was then obtained by retroorbital puncture 96 hours after treatment for determination of blood urea nitroben (BUN) levels (a standard method of screening compounds for potential renal toxicity).

| Compound Number | Relative Dose | BUN (mg/100 ml) |
|---|---|---|
| 1 | $LD_{10}$ | 31±8 |
| | $LD_{50}$ | 35±12 |
| 2 | $LD_{10}$ | 38±8 |
| | $LD_{50}$ | 50±15 |
| 3 | $LD_{10}$ | 40±7 |
| | $LD_{50}$ | 39±5 |
| 4 | $LD_{10}$ | 36±12 |
| | $LD_{50}$ | 38±10 |
| 0.09% NaCl | — | 32±2 |
| cis diamminedichloro | $LD_{10}$ | 53±19 |
| platinum(II) | $LD_{50}$ | 67±10 |

Example 13

Cis-bis-2-aminoethanesulfonato(DACH)platinum(II)

Sulfato (DACH)platinum(II), 0.202 g, was dissolved in 10 ml of water and barium-2-amino-ethanesulfonate, prepared *in situ* by the addition of $Ba(OH)_2.8H_2O$, 0.15 g, to an aqueous solution of 2-aminoethanesulfonic acid, 0.125 g, was added thereto and the reaction mixture was stirred for 30 minutes at room temperature. Barium sulfate precipitate was filtered off and the yellow filtrate was evaporated to dryness at 45—50°C under reduced pressure using a rotary evaporator. A brown yellow solid was obtained which was then washed with methanol and acetone. The product was obtained which was then washed with methanol and acetone. The product was finally dried in vacuo.

Yield = 80%.

The analytical data for the product are set forth in Table III.

Example 14

Cis-bis-L-prolinato(trans-1-DACH)platinum(II)

Sulfato (trans-1-DACH)platinum(II), (0.202 g) was dissolved in 20 ml of water and the barium salt of L-proline, prepared *in situ* by the addition of $Ba(OH)_2.8H_2O$ (0.150 g) to an aqueous solution of L-proline (0.115 g) was added thereto and the reaction mixture was stirred for 20—30 minutes at room temperature. Barium sulfate precipitate was filtered off and the yellow filtrate was evaporated to dryness at 45—50°C under reduced pressure using a rotary evaporator. A yellowish-white solid was obtained which was then washed with methanol and acetone. The product was finally dried in vacuo.

Yield = 75%.

The analytical data for the product are set forth in Table III.

Example 15

Iminodiacetato(trans-1-DACH)platinum(II)$2H_2O$

Sulfato (trans-1-DACH)platinum(II), 0.405 g, was dissolved in 50 ml of water and barium iminodiacetate prepared *in situ* by the addition of $Ba(OH)_2.8H_2O$, 0.3 g, to an aqueous solution of iminodiacetic acid, 0.133 g in 100 ml water was added thereto and the reaction mixture was stirred for 20—30 minutes at room temperature. Barium sulfate precipitate was filtered off and the yellow filtrate was evaporated to dryness at 45—50°C under reduced pressure using a rotary evaporator. A yellowish-white solid was obtained which was then washed with acetone. The product was then dried in vacuo.

Yield = 70%.

The analytical data for the product are set forth in Table III.

Example 16

Cis-bis-cyclopropanecarboxylato(DACH)platinum(II)

Sulfato (DACH)platinum(II), 0.405 g (1 mmole), was dissolved in water (20 ml) and barium cyclopropanecarboxylate, prepared *in situ* by the addition of $Ba(OH)_2.8H_2O$ (0.30 g) to an aqueous solution of cyclopropanecarboxylic acid (0.172 g) (2 mmole), was added thereto and the reaction mixture was stirred

for 20—30 minutes at room temperature. Barium sulfate precipitate was filtered off and the yellow filtrate was evaporated to dryness at 45—50°C under reduced pressure using a rotary evaporator. A brown yellow solid was obtained, which was purified with methanol. The product was finally dried in vacuo.

Yield = 70%.

The analytical data for the product are set forth in Table III.

Other complexes of the invention, i.e. bis-cyclobutanecarboxylate, bis-cyclopentanecarboxylato, bis-cyclopentenecarboxylato, bis-cyclohexanecarboxylato, bis-cyclohexenecarboxylato, cyclopropane-1,1-dicarboxylato, trans-DL-1,2-cyclopentanecarboxylato, cyclohexane-1,1-diacetato, bicinato, chloroethanesulfonato, N-(2-hydroxyethyl)-iminodiaceto, N-(2-acetamido)-iminodiacetato, 3,4-furanedicarboxylato, trans-1,2-cyclopropane dicarboxylato, trans-1,2-cyclobutanedicarboxylato, trans-1,2-cyclopentane dicarboxylato and trans-1,2-cyclohexanedicarboxylato were prepared in an analogous manner to the above mentioned method using stoichiometric amounts (ca/1mmole) sulfato (DACH) platinum (II), and the respective barium salts (prepared *in situ*) i.e. cyclobutanecarboxylate, cyclopentanecarboxylate, cyclopentenecarboxylate, cyclohexanecarboxylate, cyclohexenecarboxylate, cyclopropane-1,1-dicarboxylate, trans-DL-1,2-cyclopentanedicarboxylate, cyclohexane-1,1-diacetate, bicinate, chloroethanesulfonate, N-(2-hydroxyethyl)iminodiacetate, N-(2-acetamido)-iminodiacetate, 3,4-furanediacarboxylate, trans-1,2-cyclopropanedicarboxylate, trans-1,2-cyclobutanedicarboxylate, trans-1,2-cyclopentanedicarboxylate and trans-1,2-cyclohexanedicarboxylate.

TABLE III

| Compound | Analytical Data | | | | | |
|---|---|---|---|---|---|---|
| | Found (%) | | | Calculated (%) | | |
| No | C | H | N | C | H | N |
| 15 | 41.23 | 6.93 | 5.46 | 41.30 | 6.54 | 4.82 |
| 16 | 36.58 | 5.75 | 5.03 | 36.55 | 5.71 | 5.33 |
| 17 | 41.20 | 5.47 | 4.64 | 41.56 | 5.89 | 4.85 |
| 18 | 38.82 | 5.29 | 5.04 | 39.06 | 6.14 | 5.06 |
| 19 | 37.77 | 5.52 | 4.97 | 37.95 | 5.62 | 4.92 |
| 20 | 33.43 | 4.58 | 5.45 | 33.50 | 4.72 | 6.01 |
| 21 | 27.95 | 5.16 | 8.58 | 27.96 | 4.87 | 8.89 |
| 22 | 33.40 | 5.31 | 5.48 | 33.79 | 5.23 | 5.63 |
| 23 | 36.61 | 5.72 | 5.21 | 36.55 | 5.71 | 5.33 |
| 24 | 20.71 | 5.05 | 9.51 | 20.86 | 4.86 | 9.73 |
| 25 | 25.31 | 5.32 | 11.64 | 25.25 | 5.05 | 11.78 |
| 26 | 18.26 | 3.91 | 3.78 | 18.46 | 3.69 | 4.30 |
| 27 | 33.98 | 5.43 | 8.33 | 34.00 | 5.98 | 8.81 |
| 28 | 25.14 | 4.37 | 8.59 | 25.20 | 4.83 | 8.82 |
| 29 | 33.41 | 5.76 | 9.47 | 33.50 | 5.93 | 9.77 |
| 30 | 34.65 | 4.94 | 7.99 | 34.77 | 4.89 | 7.60 |
| 31 | 38.50 | 5.20 | 7.31 | 38.47 | 4.71 | 7.92 |
| 32 | 27.96 | 5.15 | 7.62 | 27.69 | 5.19 | 8.07 |
| 33 | 27.96 | 4.43 | 5.57 | 27.90 | 4.65 | 5.92 |
| 34 | 31.64 | 4.83 | 5.85 | 31.93 | 4.43 | 6.21 |
| 35 | 28.34 | 5.22 | 10.47 | 28.45 | 4.54 | 11.00 |
| 36 | 33.86 | 5.62 | 5.40 | 33.12 | 5.12 | 5.52 |

In Vitro Cytotoxicity

The following compounds were tested for *in vitro* cytotoxicity according to the protocol discussed in Example 7.

| Compound No. | $ID_{50}$(μg/ml) |
|---|---|
| 15 | 0.5 |
| 16 | 0.6 |
| 17 | 1.0 |
| 18 | 0.3 |
| 19 | 1.3 |
| 20 | 0.4 |
| 21 | 1.6 |
| 22 | 0.3 |
| 23 | 0.4 |
| 24 | 3.1 |
| 25 | 2.5 |
| 26 | 0.3 |
| 27 | 1.2 |
| 28 | 3.0 |
| 29 | 0.8 |
| 30 | 1.0 |
| 31 | 2.3 |
| 32 | 3.0 |
| 33 | 0.4 |
| 34 | 0.5 |
| 35 | 0.7 |

In Vivo Efficacy Studies — Single Dose

The following compounds were tested for *in vivo* oncolytic activity following a single i.p. injection of the drug according to the protocol described in Example 9.

| Compound No. | Dose (mg/kg) | T/C | LTS |
|---|---|---|---|
| 18 | 50 | 163 | — |
| | 25 | 178 | — |
| | 12.5 | 161 | — |
| 20 | 50 | 183 | — |
| | 25 | 159 | — |
| | 12.5 | 158 | — |
| 21 | 50 | Toxic | — |
| | 25 | 185 | — |
| | 12.5 | 161 | — |
| 22 | 50 | 199 | 1/6 |
| | 25 | 176 | — |
| | 12.5 | 252 | 1/6 |
| 23 | 50 | 145 | — |
| | 25 | 154 | — |
| | 12.5 | 149 | — |
| 19 | 50 | 157 | — |
| | 25 | 158 | — |
| | 12.5 | 185 | — |
| 24 | 50 | 153 | — |
| | 25 | 134 | — |
| | 12.5 | 109 | — |
| 25 | 50 | 158 | — |
| | 25 | 131 | — |
| | 12.5 | 128 | — |
| 26 | 50 | Toxic | — |
| | 25 | 144 | — |
| | 12.5 | 188 | — |
| 27 | 50 | 160 | — |
| | 25 | 151 | — |
| | 12.5 | 165 | — |
| 29 | 50 | 149 | — |
| | 25 | 212 | 1/6 |
| | 12.5 | 179 | — |
| 30 | 50 | 108 | — |
| | 25 | 156 | — |
| | 12.5 | 172 | — |
| 31 | 50 | 158 | — |
| | 25 | 170 | — |
| | 12.5 | 123 | — |
| 15 | 100 | 170 | — |
| | 50 | 140 | — |
| 17 | 100 | 105 | — |
| | 50 | 165 | — |
| | 25 | 132 | — |

In Vivo Efficacy Studies — Multiple Dose

The following complexes were tested for *in vivo* oncolytic activity following 3 i.p. injections (days 1, 5, 9) according to the protocol described in Examples 9 and 10.

| Compound No. | Dose (mg/kg) | T/C | LTS |
|---|---|---|---|
| 18 | 25 | 253 | — |
| | 12.5 | 246 | — |
| | 6.25 | 202 | — |
| 19 | 25 | 300 | 1/6 |
| | 12.5 | 356 | 1/6 |
| | 6.25 | 189 | — |
| 24 | 100 | 295 | 2/6 |
| | 50 | 198 | 1/6 |
| 30 | 25 | 242 | 1/6 |
| | 12.5 | 298 | 5/6 |
| | 6.25 | 246 | 3/6 |
| 31 | 25 | 240 | 2/6 |
| | 12.5 | 287 | 1/6 |
| | 6.25 | 277 | — |

T/C calculated for 30 day observation period.
Long term survivor data represents survivors/treated 50 days after treatment.

The platinum complexes of the invention may be compounded with suitable pharmaceutically acceptable carriers and administered orally, intramuscularly, topically, etc. It is preferred, however, to combine the complex with suitable media, e.g., 5% dextrose, klucel, water, etc., for intravenous administration. Care should be taken, however, to avoid the use of saline as an i.v. medium.

Those skilled in the art will be aware of suitable carriers for the complexes of the invention suitable for formulations into capsules, tablets, powders, ointments, pellets, etc.

The complexes may also be administered in combination with other anti-tumor agents in a combined chemotherapeutic regimen.

The amount of complex included in the pharmaceutical composition and the dosage of complex utilized in the method of treatment of the invention will vary depending in each case upon the condition of the patient, the nature of the tumor undergoing treatment, the anti-tumor activity of the complex, the toxicity and solubility characteristics thereof, etc. Generally, however, an amount of platinum complex ranging from about 25 to about 200 mg/kg is adequate for most applications.

**Claims**

1. A water-soluble square planar *cis*-platinum (II) four-coordinate complex having the formula:

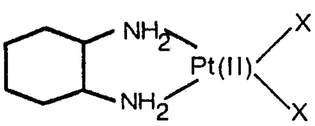

or

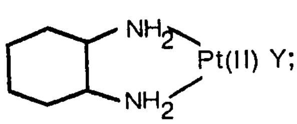

;

wherein:

X is a monovalent anion selected from the group consisting of monosaccharate, saccharate-4-lactone, shikimate, isethionate, 2-aminoethylsulfate, azetidinecarboxylate, proline, hydroxyproline, pipecolinate, cyclopropanecarboxylate, cyclobutanecarboxylate, cyclopentenecarboxylate, cyclohexanecarboxylate, cyclohexenecarboxylate, bicine, glycine, 2-amino-ethanesulfonate, 2-chloroethanesulfonate and

Y is a divalent anion selected from the group consisting of iminodiacetate, isocitrateactone, furanedicarboxylate, cyclopropane-1,1-dicarboxylate, isocitratomonoethylester, N-methyliminodiacetate, N-(2-hydroxyethyl)-iminodiacetate, N-benzyliminodiacetate, N-phenyliminodiacetate, N-(2-acetamido)-iminodiacetate, cyclohexane-1,1-diacetate, trans-1,2-cyclopropanedicarboxylate, trans-1,2-cyclobutanedicarboxylate, trans-1,2-cyclopentanedicarboxylate and trans-1,2-cyclohexanedicarboxylate.

2. A platinum complex of claim 1 which is selected from:

A.

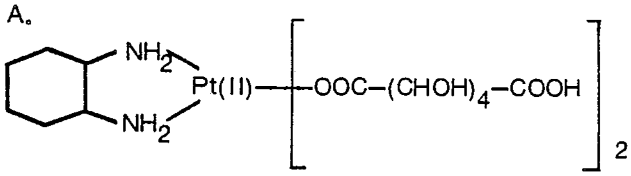

[bis-monosaccharato]

B.

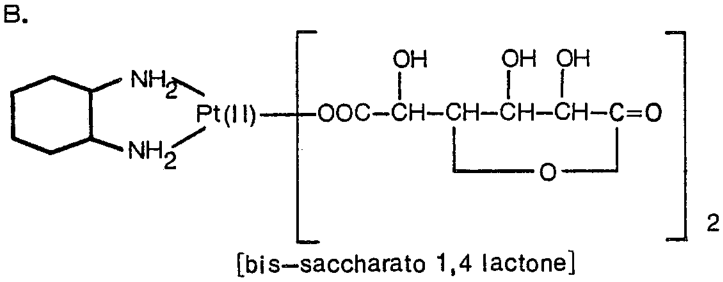

[bis–saccharato 1,4 lactone]

C.

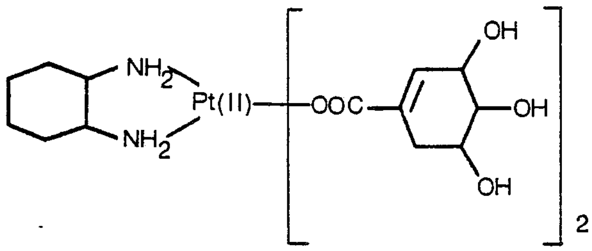

[bis–shikimato]

D.

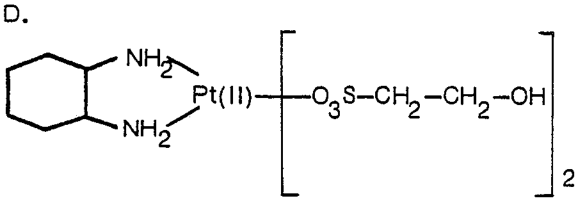

[bis–isethionato]

E.

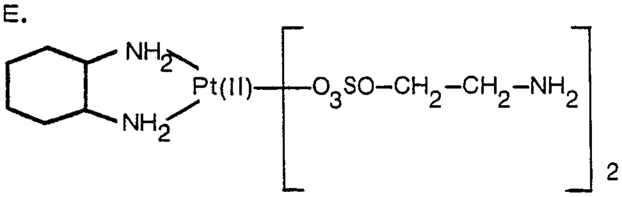

[bis–2–aminoethylsulfato]

F.

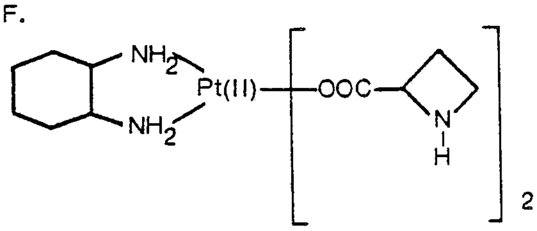

[bis–azetidinecarboxylato]

G.

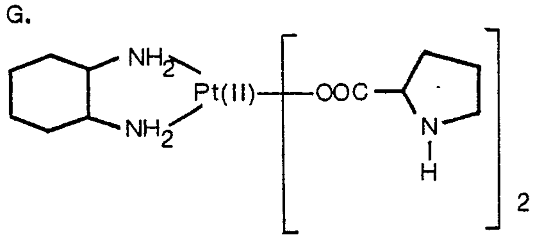

[bis–prolinato]

H.

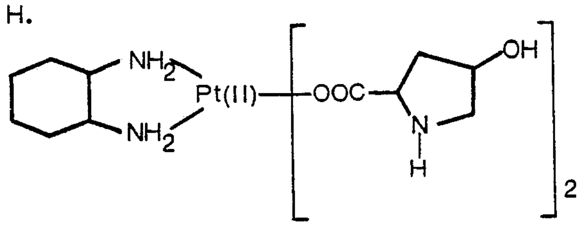

[bis–hydroxyprolinato]

I.

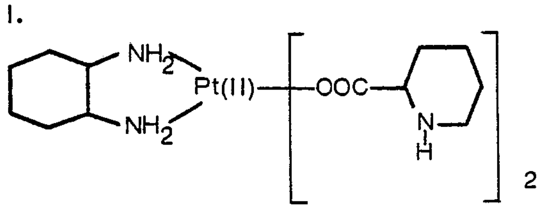

[bis–pipecolinato]

J.

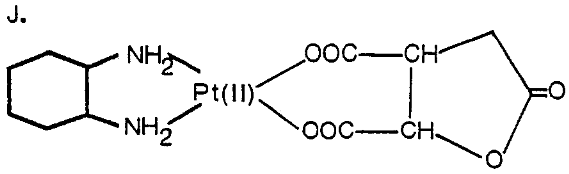

[isocitratolactone]

K.

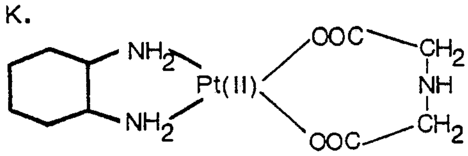

[Iminodiacetato]

L.

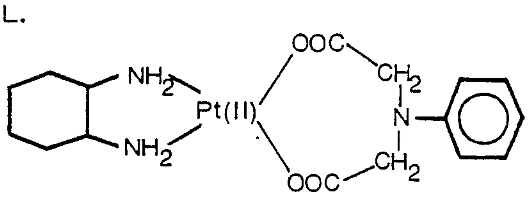

[N–phenyliminodiacetato]

M.

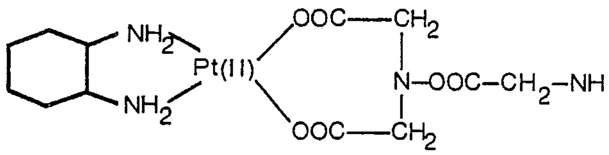

[N-(2-acetamido)-iminodiacetato]

N.

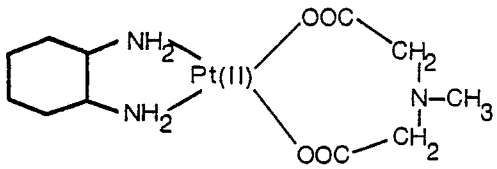

[N–methyliminodiacetato]

O.

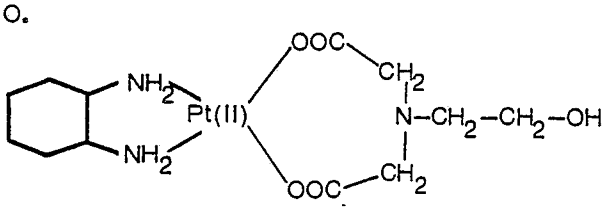

[N–(2–hydroxyethyl)iminodiacetato]

P.

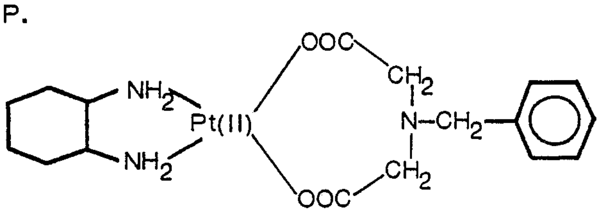

[N–benzyliminodiacetato]

Q.

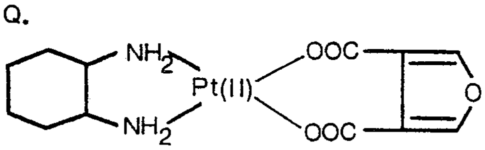

[furanedicarboxylate]

R.

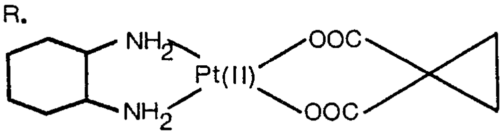

[cyclopropanedicarboxylato–]

S.

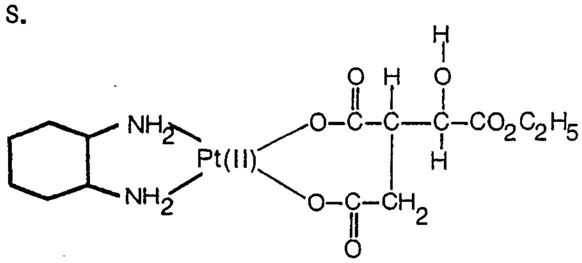

[isocitratomonoethylester]

T.

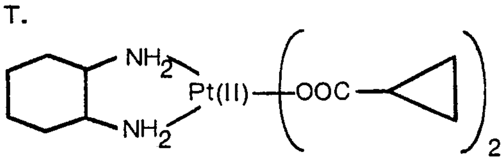

[bis–cyclopropanecarboxylato]

U.

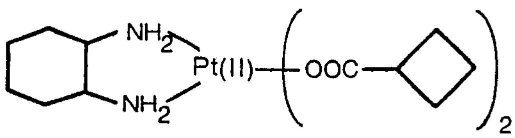

[bis–cyclobutanecarboxylato]

V.

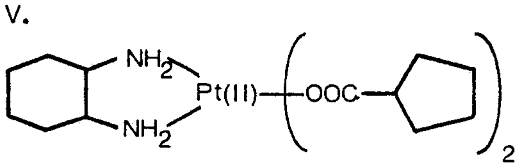

[bis–cyclopentanecarboxylato]

W.

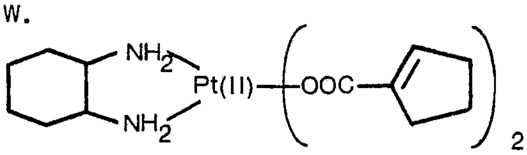

[bis–cyclopentenecarboxylato]

X.

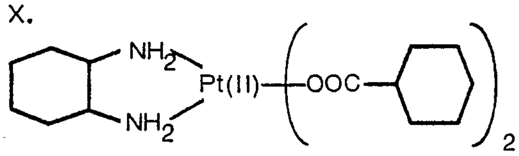

[bis–cyclohexanecarboxylato]

Y.

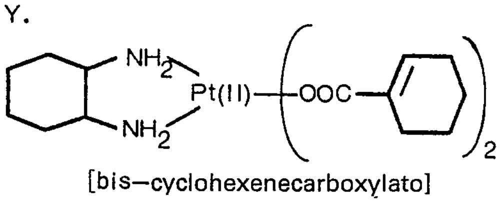

[bis–cyclohexenecarboxylato]

Z.

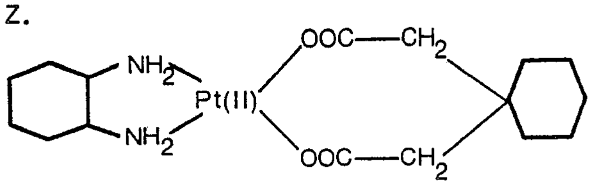

[cyclohexane–1,1–diacetato]

Z1.

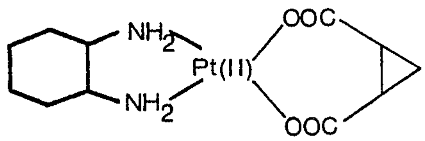

[1,2–cyclopropanedicarboxylato]

Z2.

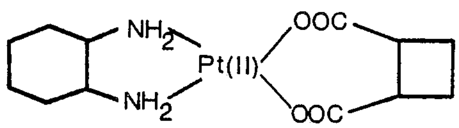

[trans–1,2–cyclobutanedicarboxylato]

Z3.

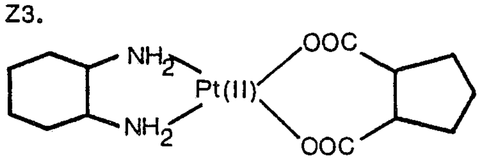

[trans–1,2–cyclopentanedicarboxylato]

Z4.

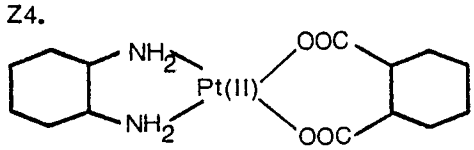

[trans–1,2–cyclohexanedicarboxylato]

Z5.

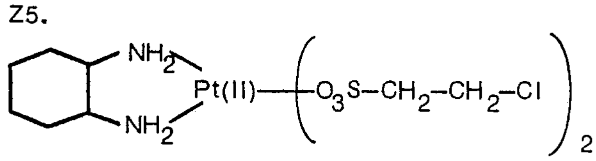

[bis–2–chloroethanesulfonato]

Z6.

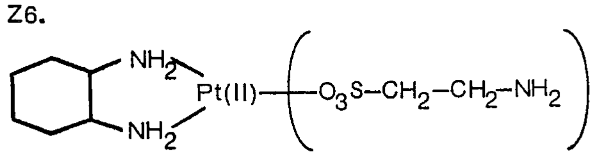

[bis–2–aminoethanesulfonato]

Z7.

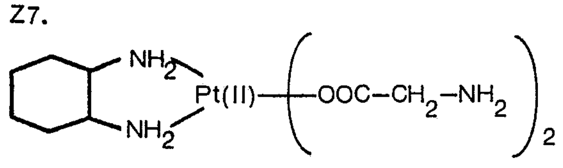

[bis–glycinato]

Z8.

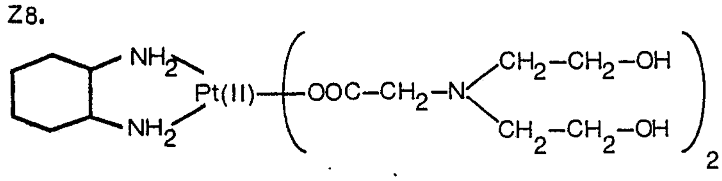

[bis–bicinato]

3. A pharmaceutical composition in unit dosage form suitable for administration to an animal afflicted with tumor cells sensitive to a platinum complex of any of the preceding claims comprising a therapeutically anti-tumor effective amount of said platinum complex and a pharmaceutically acceptable carrier therefor.

4. A method for the preparation of a complex of claim 1 comprising:

a) reacting a water-soluble haloplatinate (II) in an aqueous medium with DACH to produce a di-lalo-(DACH)-platinum (II);

b) reacting said product of step a) with a soluble sulfate salt in an aqueous medium to produce a sulfato-(DACH)-platinum (II);

c) reacting the product of step b) with a soluble salt of X or Y to produce said complex of claim 1, and

d) recovering said complex.

**Patentansprüche**

1. Wasserlöslicher quadratisch-planarer *cis*-Platin(II)-vier-Koordinationskomplex mit der Formel

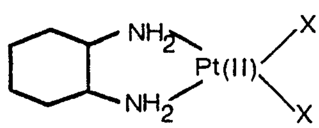

oder

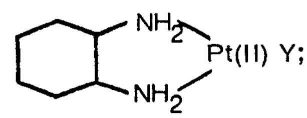

worin bedeuten:

X ein einwertiges Anion, ausgewählt aus der Gruppe bestehend aus Monosaccharat, Saccharat-4-lacton, Shikimat, Isethionat, 2-Aminoethylsulfat, Azetidincarboxylat, Prolin, Hydroxyprolin, Pipecolinat, Cyclopropancarboxylat, Cyclobutancarboxylat, Cyclopentancarboxylat, Cyclopentencarboxylat, Cyclohexancarboxylat, Cyclohexancarboxylat, Bicin, Glycin, 2-Aminoethanesulfonat, 2-Chlorethanesulfonat und

Y ein zweiwertiges Anion, ausgewählt aus der Gruppe bestehend aus Iminodiacetat, Isocitratlacton, Furandicarboxylat, Cyclopropan-1,1-dicarboxylat, Isocitratmonoethylester, N-Methyliminodiacetat, N-(2-Hydroxyethyl)iminodiacetat, N-Benzyliminodiacetat, N-Phenyliminodiacetat, N-(2-Acetamido)-iminodiacetat, Cyclohexan-1,1-diacetat, trans-1,2-Cyclopropandicarboxylat, trans-1,2-Cyclobutandicarboxylat, trans-1,2-Cyclopentandicarboxylat und trans-1,2-Cyclohexandicarboxylat.

2. Platikomplex gemäß Anspruch 1, ausgewählt aus

A.

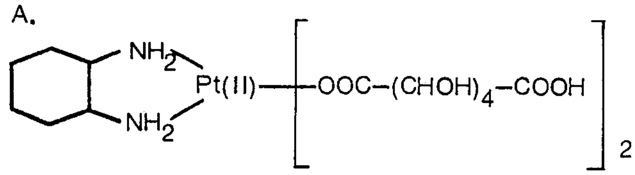

[bis–Monoaccharato]

B.

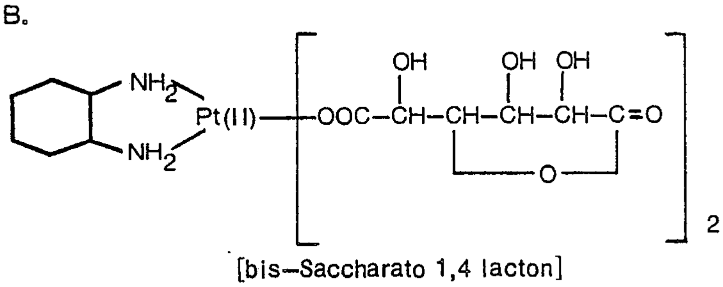

[bis–Saccharato 1,4 lacton]

C.

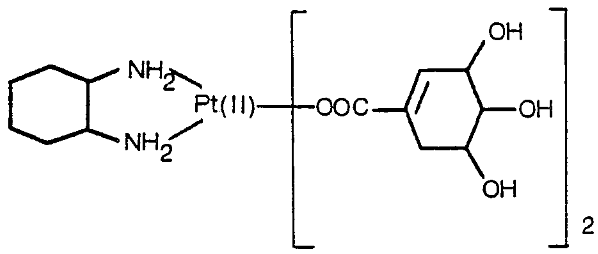

[bis–Shikimato]

D.

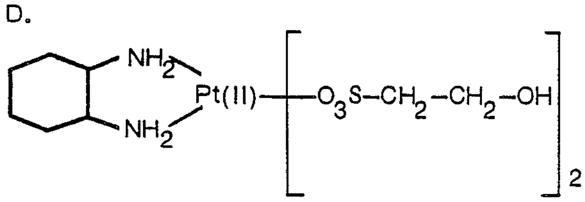

[bis–Isethianato]

E.

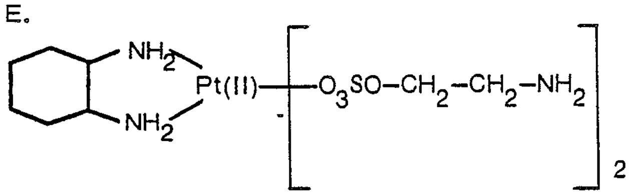

[bis–2–Aminoethylsulfato]

F.

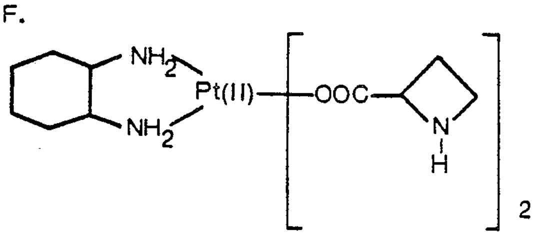

[bis–Azetidincarboxylato]

G.

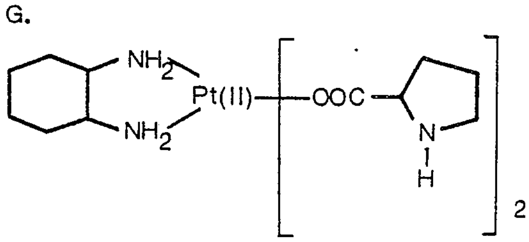

[bis–Prolinato]

H.

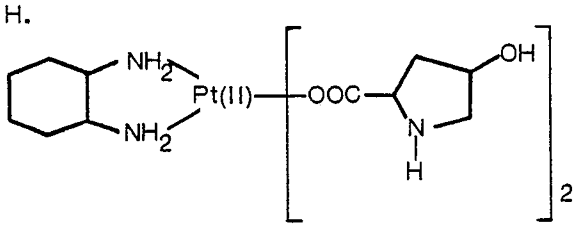

[bis–Hydroxyprolinato]

I.

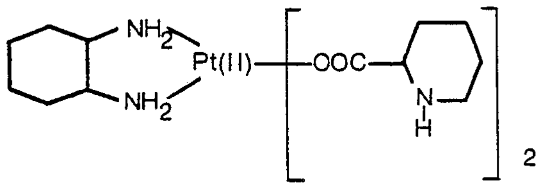

[bis–Pipecolinato]

J.

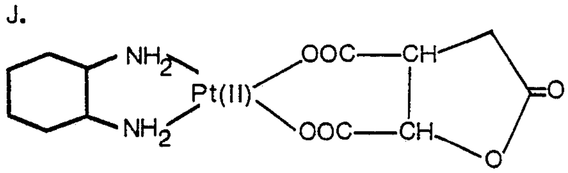

[Isocitratolacton]

K.

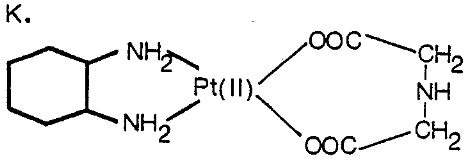

[Iminodiacetato]

L.

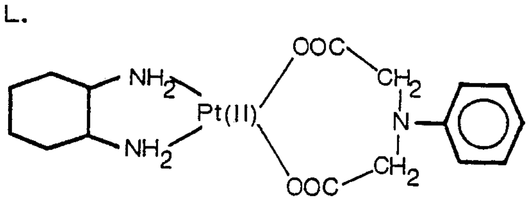

[N–Phenyliminodiacetato]

M.

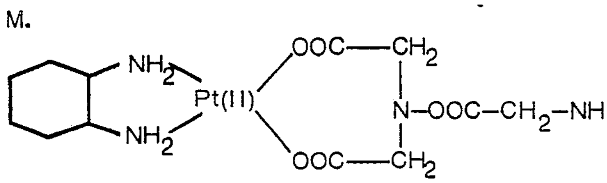

[N–(2–Acetamido)–iminodiacetato]

N.

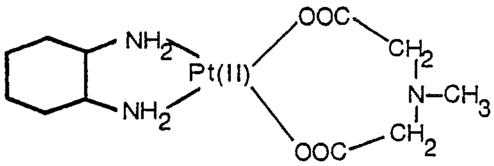

[N–Methyliminodiacetato]

O.

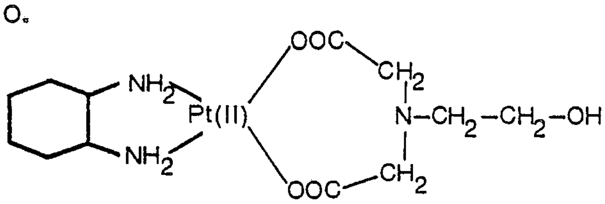

[N–(2–Hydroxyethyl)iminodiacetato]

P.

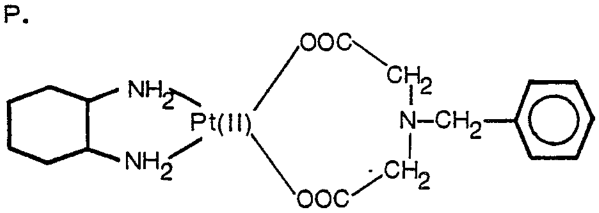

[N–Benzlyiminodiacetato]

Q.

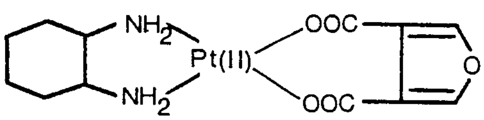

[Furandicarboxylato]

R.

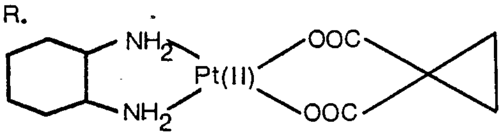

[Cyclopropandicarboxylato]

S.

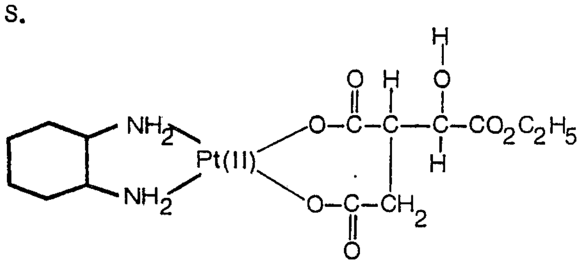

[Isocitratomonoethylester]

T.

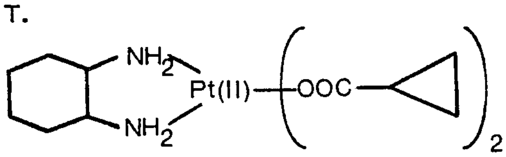

[bis–Cyclopropancarboxylato]

U.

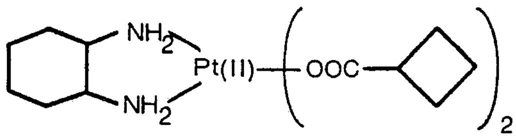

[bis–Cyclobutancarboxylato]

V.

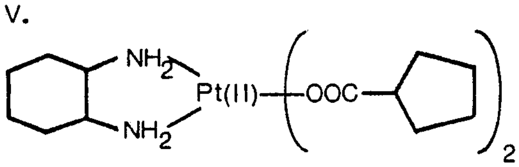

[bis–Cyclopentancarboxylato]

W.

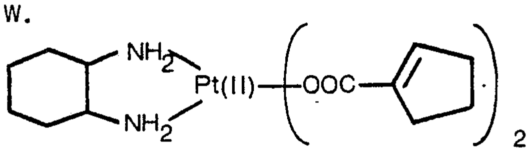

[bis–Cyclopentencarboxylato]

X.

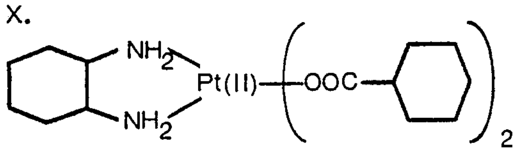

[bis–Cyclohexancarboxylato]

Y.

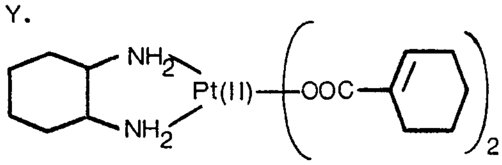

[bis–Cyclohexencarboxylato]

Z.

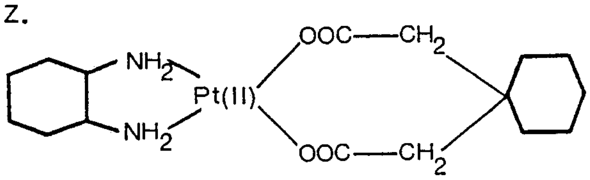

[Cyclohexan–1,1–diacetato]

Z1.

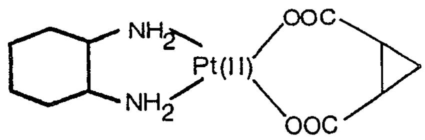

[1,2–Cyclopropandicarboxylato]

Z2.

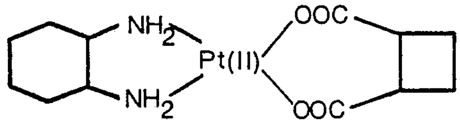

[Trans–1,2–cyclobutandicarboxylato]

Z3.

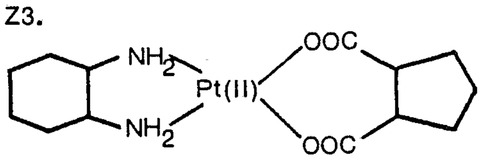

[Trans–1,2–cyclopentandicarboxylato]

Z4.

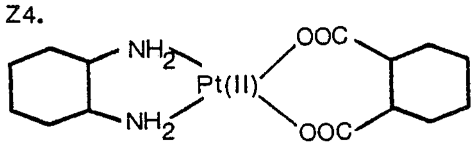

[Trans–1,2–cyclohexandicarboxylato]

Z5.

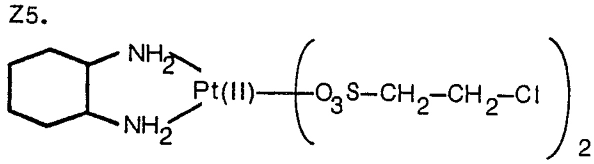

[bis–2–Chloroethansulfonato]

Z6.

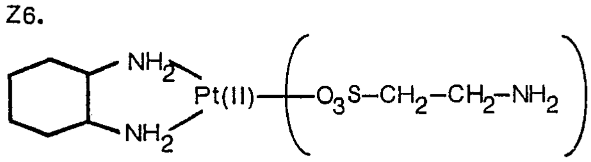

[bis–2–Aminoethansulfonato]

Z7.

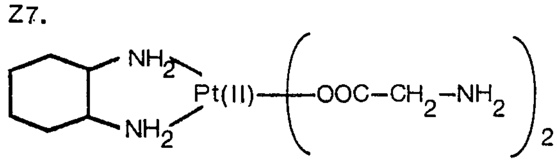

[bis–Glycinato]

Z8.

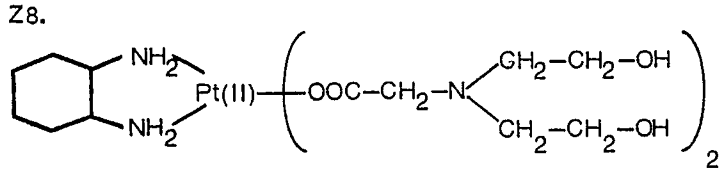

[bis–Bicinato]

3. Pharmazeutische Zusammensetzung in einer Einheitsdosierungsform, die zur Verabreichung an ein Geschöpf, das mit — gegenüber einem Platinkomplex gemäß einem der vorhergehenden Ansprüche — empfindlichen Turmorzellen befallen ist, geeignet ist, umfassend eine therapeutisch Antitumor-wirksame Menge des Platinkomplexes und einen pharmazeutisch annehmbaren Träger dafür.

4. Verfahren zur Herstellubg eines Komplexes gemäß Anspruch 1 umfassend:

(a) Umsetzen eines wasserlöslichen Haloplatinats(II) in einem wäßrigen Medium mit DACH unter Ausbildung eines di-Halo-(DACH)-platin (II);

(b) Umsetzen des Produktes gemäß Stufe (a) mit einem löslichen Sulfatsalz in einem wäßrigen Medium unter Ausbildung von Sulfato(DACH)-platin (II);

(c) Umsetzen des Produktes gemäß Stufe (b) mit einem löslichen Salz von X oder Y unter Ausbildung des Komplexes gemäß Anspruch 1, und

(d) Gewinnen des Komplexes.

**Revendications**

1. Complexe de coordinence 4 de *cis*-platine (II), en carré dans un plan, soluble dans l'eau, présentant la formule:

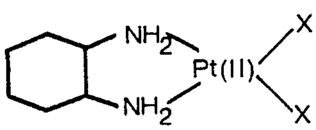

on

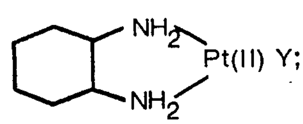

où:

X est un anion monovalent choisi dans le groupe constitué par monosaccharate, saccharate-4-lactone, shikimate, iséthionate, 2-aminoéthylsulfate, azétidinecarboxylate, proline, hydroxyproline, pipécolinate, cyclopropanecarboxylate, cyclobutanecarboxylate, cyclopentanecarboxylate, cyclopentènecarboxylate, cyclohexanecarboxylate, cyclohexènecarboxylate, bicine, glycine, 2-aminoéthanesulfonate, 2-chloroéthanesulfonate, et

Y est un anion divalent choisi dans le groupe constitué par iminodiacétate, isocitratelactone, furannedicarboxylate, cyclopropane-1,1-dicarboxylate et isocitratomonoéthylester, N-méthyliminodiacétate, N-(2-hydroxyéthyl)-iminodiacétate, N-benzyliminodiacétate, N-phényliminodiacétate, N-(2-acétamido)-iminodiacétate, cyclohexane-1,1-diacétate, trans-1,1,2-cyclopropanedicarboxylate, trans-1,2-cyclobutanedicarboxylate, trans-1,2-cyclopentanedicarboxylate et trans-1,2-cyclohexanedicarboxylate.

2. Complexe du platine selon la revendication 1, qui est choisi parmi:

A.

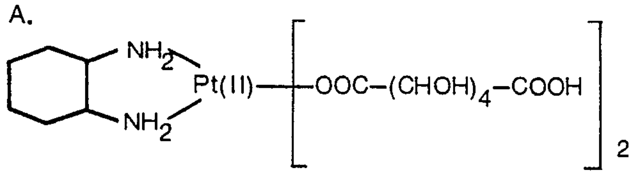

[bis–monosaccharato]

B.

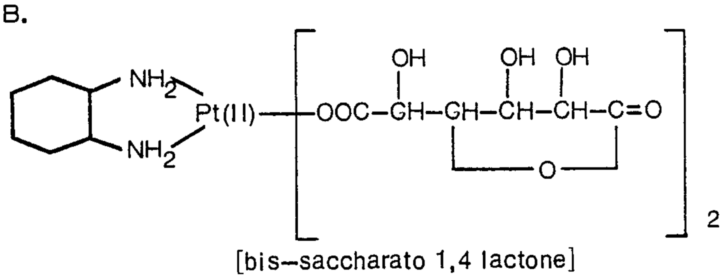

[bis–saccharato 1,4 lactone]

C.

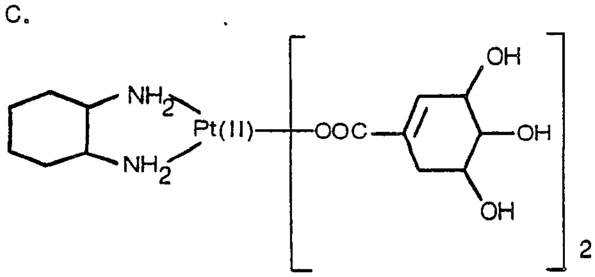

[bis–shikimato]

D.

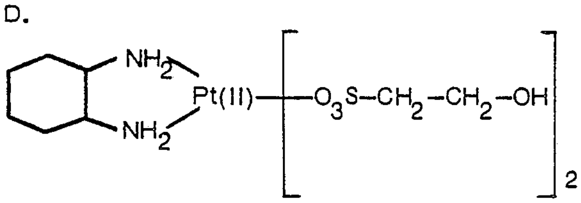

[bis–iséthionato]

E.

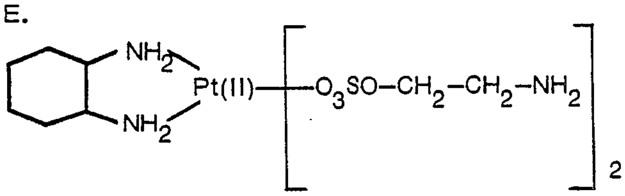

[bis–2–aminoéthylsulfato]

F.

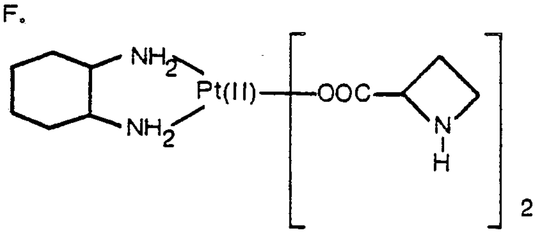

[bis–azétidinecarboxylato]

G.

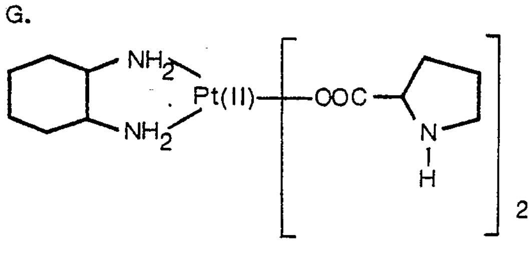

[bis–prolinato]

H.

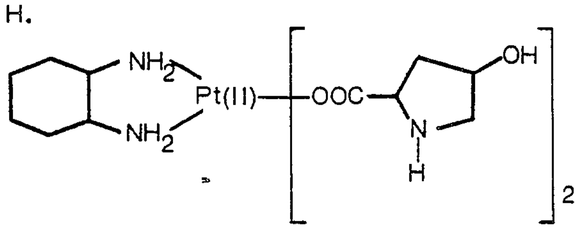

[bis–hydroxyprolinato]

I.

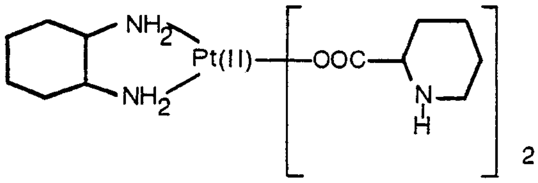

[bis–pipécolinato]

J.

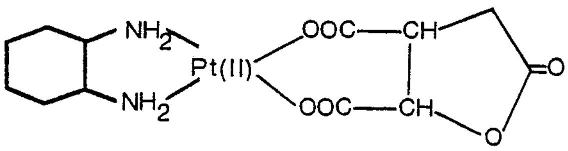

[isocitratolactone]

K.

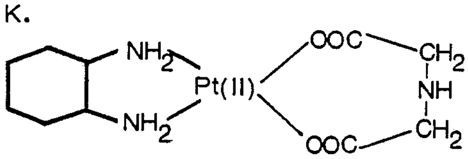

[iminodiacétato]

L.

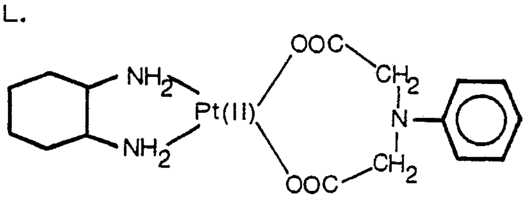

[N–phényliminodiacétato]

M.

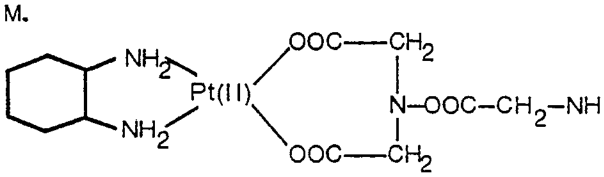

[N-(2-acétamido)-iminodiacéato]

N.

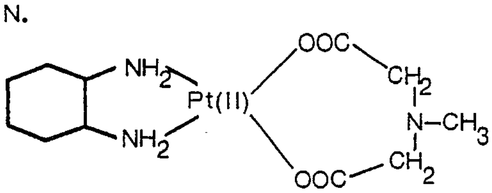

[N–méthyliminodiacétato]

O.

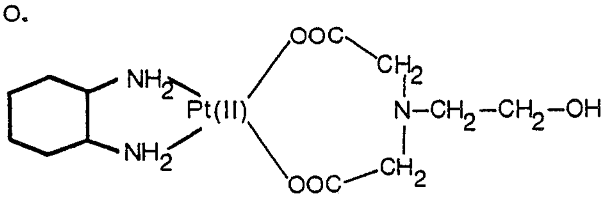

[N—(2—hydroxyéthyl)iminodiacétato]

P.

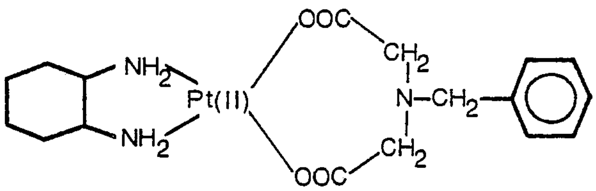

[N—benzyliminodiacétato]

Q.

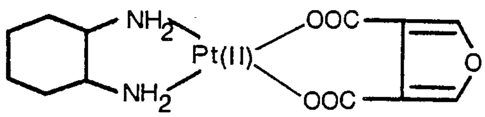

[furannedicarboxylato]

R.

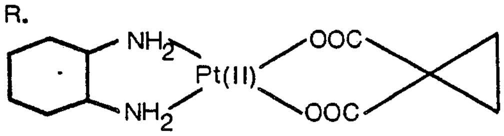

[cyclopropanedicarboxylato]

S.

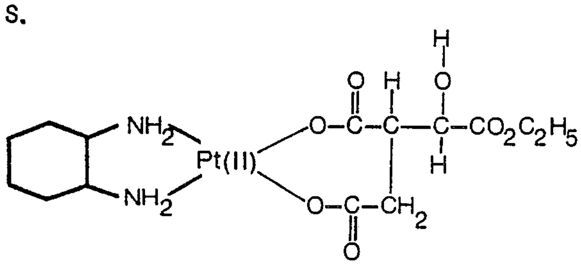

[isocitratomonoéthylester]

T.

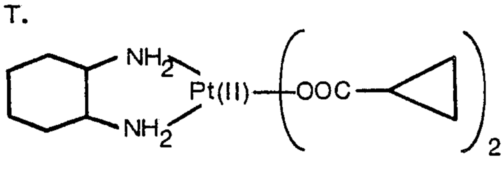

[bis—cyclopropanecarboxylato]

U.

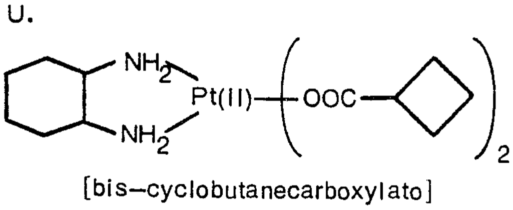

[bis—cyclobutanecarboxylato]

V.

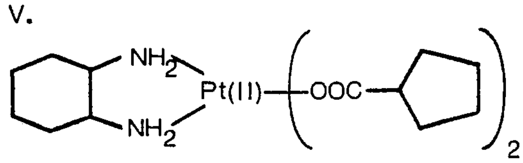

[bis–cyclopentanecarboxylato]

W.

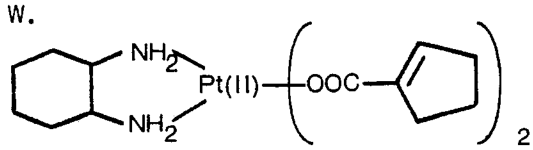

[bis–cyclopentènecarboxylato]

X.

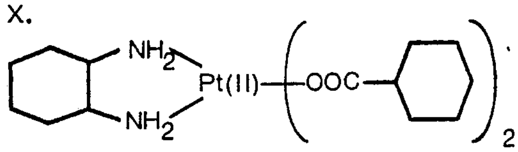

[bis–cyclohexanecarboxylato]

Y.

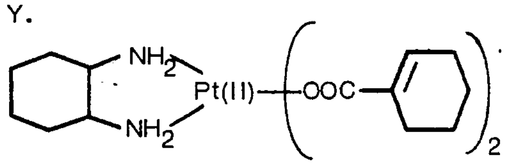

[bis–cyclohexènecarboxylato]

Z.

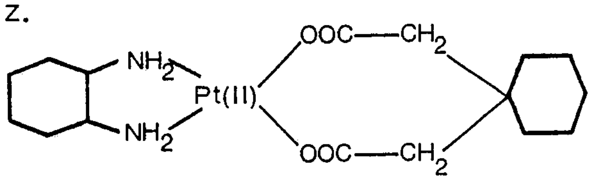

[cyclohexane–1,1–diacétato]

Z1.

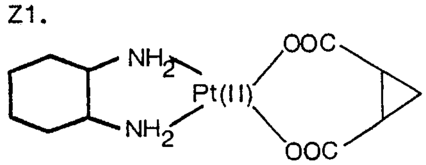

[1,2–cyclopropanedicarboxylato]

Z2.

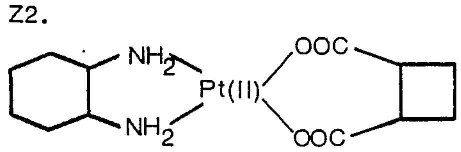

[trans–1,2–cyclobutanedicarboxylato]

Z3.

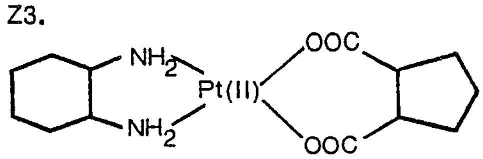

[trans–1,2–cyclopentanedicarboxylato]

Z4.

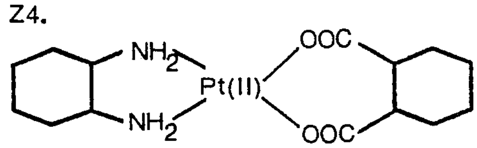

[trans–1,2–cyclohexanedicarboxylato]

Z5.

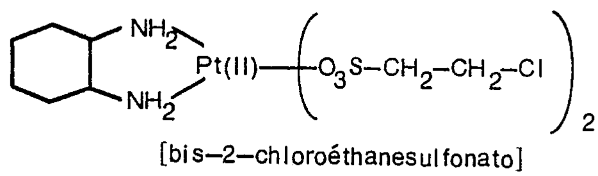

[bis–2–chloroéthanesulfonato]

Z6.

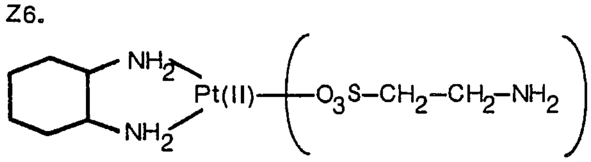

[bis–2–aminoéthanesulfonato]

Z7.

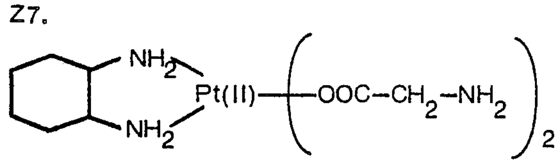

[bis–glycinato]

Z8.

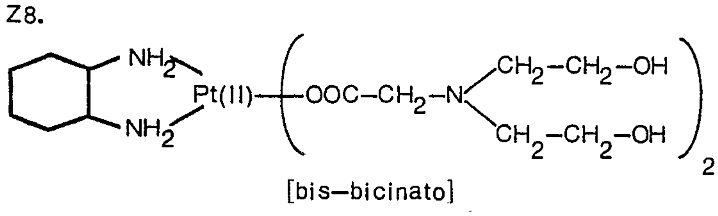

[bis–bicinato]

3. Composition pharmaceutique, se présentant sous forme de dosages unitaires, convenant pour être administrée à un animal affligé de cellules tumorales sensibles à un complexe du platine tel que défini à l'une des revendications précédentes, ladite composition comprenant une quantité thérapeutiquement efficace contre la tumeur dudit complexe du platine, et un support pharmaceutiquement acceptable dudit complexe.

4. Procédé de fabrication d'un complexe tel que défini à la revendication 1, consistant à:

a) faire réagir un haloplatinate (II) soluble dans l'eau dans un milieu aqueux, avec DACH, pour obtenir un di-halo-(DACH)-platine (II);

b) faire réagir le produit précité de l'étape a) avec un sel sulfate soluble, dans un milieu aqueux, pour obtenir du sulfato (DACH)-platine (II);

c) faire réagir le produit de l'étape b) avec un sel soluble de X ou Y, pour obtenir ledit complexe de la revendication 1; et

d) récupérer ledit complexe.